# EUROPEAN PATENT APPLICATION

(11) **EP 3 138 561 A1**
(43) Date of publication of application: **08.03.2017**
(21) Application number: 15777294.8
(22) Date of filing: 10.04.2015
(51) Int. Cl.: A61K 31/14, A61P 11/00, A61P 31/04, A61P 31/02

(54) **USE OF DECAMETHOXINE AS AN ANTIMICROBIAL AGENT AGAINST BACTERIA OF THE GENUS BURKHOLDERIA OR OF THE SPECIES PSEUDOMONAS AERUGINOSA**

(30) Priority: 10.04.2014 UA 2014003770 U
(71) Applicant: Gumeniuk, Mykola Ivanovych, Kiev 03110 (UA)
(72) Inventor: DERKACH, Nataliia Mykolaivna, Kiev 03110 (UA); GOROVENKO, Nataliia Grygorivna, Kiev 01004 (UA)
(74) Representative: Anohins, Vladimirs
(86) International application number: PCT/UA2015/000032
(87) International publication number: WO 2015/156753

(57) **Abstract**

Technical solution refers to the novel use of decamethoxine.

The novel use of decamethoxine is concerned with its use as antimicrobial agent against bacteria of the Burkholderia genus or bacteria of the *Pseudomonas aeruginosa* species.

The technical result achieved by the present technical solution: new decamethoxine property found (antimicrobial action against bacteria of the Burkholderia genus or of the *Pseudomonas aeruginosa* species) makes possible the development of more efficient drugs for the treatment of diseases caused by bacteria of the Burkholderia genus or bacteria of the *Pseudomonas aeruginosa* species, and adds to the arsenal of drugs for the treatment of diseases caused by bacteria of the Burkholderia genus or bacteria of the *Pseudomonas aeruginosa* species.

## Description

### Field of invention

The technical solution concerns novel application of decamethoxine.

### Prior art

Burkholderia is a genus of straight or slightly curved Gram-negative bacteria, which have been singled out into independent genus from the Pseudomonas genus on the basis of molecular genetic studies. It is represented by 20 species, mostly known for their pathogenic representatives, such as *Burkholderia mallei,* causative agent of glanders, a disease of horse and animals, *Burkholderia pseudomallei,* causative agent of melioidosis, and *Burkholderia cepacia,* causative agent of lung infections in patients with cystic fibrosis. They are motile, with the exception of *B*. *mallei,* due to the presence of several polar flagellae. Oxybionts. Widespread in nature in soil, in water, and on the plants.

Pathogenic Burkholderia belong to causative agents of dangerous infections and are potential bioterrorism agents. Causative agents of glanders and melioidosis cause severe infectious diseases with aspiration, alimentary and contact mechanisms of infection. The infection takes place as a result of contact with infected water, food, and soil. Spreading of causative agents is possible owing to importation of sick animals, aquarium fishes, soil, water, as well as food products contaminated with these disease agents. The interest of researchers to Burkholderia remains alive until now due to a number of reasons.

Causative agent of melioidosis, *Burkholderia pseudomallei,* which is capable of causing severe diseases in humans and animals, is widespread in nature, mainly in South-East Asia, North Australia, some regions of Africa, North and Central America. Although historically melioidosis is considered a relatively rare disease, its incidence has recently increased in a number of countries owing to a surge in tourist visits to world regions endemic to the given infection and improvements in the laboratory diagnostic methods. Also the attention is drawn to spreading of causative agent of glanders *Burkholderia mallei,* which is capable of causing acute and chronic diseases in humans professionally engaged in animal attendance.

At present, the concern has substantially increased on the possibility of B. *pseudomallei* and *B. mallei* use as potential bioterrorism agents. In the course of epidemiological monitoring of environmental objects, there have been isolated causative agents *B*. *cepacia-complex* and *Burkholderia thailandensis,* which are phenotypically and genotypically related to *B. pseudomallei* and *B*. *mallei.*

All known species of Burkholderia bacteria are characterized by high immunity to antibacterial agents, they all have in common high resistance to antibacterial preparations, including antibiotics. Such natural resistance ensures their survivability in the environment and creates difficulties in treating diseases.

At present, the infections control worldwide assumes special character, since expansion of international contacts, tourism and international trade create great possibilities for spreading rare endemic species of pathogenic microorganisms, including causative agents of glanders and melioidosis belonging to the Burkholderia genus, from their natural seats. Recently, interest in diagnostics and treatment of said diseases has grown significantly in a number of countries in Europe and America, because features of causative agents of the Burkholderia genus allow them to create relatively stable secondary seats of infection when these causative agents come to different climatic conditions. Moreover, it is noted that diseases in humans often proceed in severe form and are hard to treat. Thus, only in Khon Kaen (Thailand) hospital 220 patients have been diagnosed with melioidosis in 3.5 years. At that, in 127 patients the disease had proceeded with phenomena of septicemia, lethality in this group reaching 90%, despite the treatment conducted with modern antibiotics.

*B. cepacia* and *B*. *thailandensis* are bacteria genetically and immunologically close to causative agent of glanders and melioidosis, but at the same time non-pathogenic to humans and animals. *B*. *cepacia* may cause opportunistic diseases in patients with immunodeficient conditions, and is especially dangerous in mucoviscidosis.

Existence of natural bacteria, genetically and immunologically close to causative agents of glanders and melioidosis, which at the sane time are non-pathogenic to humans and animals, is a long-established fact. However, only in the last decade, due to heightened interest to the problem of melioidosis incidence worldwide, isolation and studies of biochemical characteristics of Burkholderia encompassed the vast number of cultures obtained from environmental objects of endemic regions by using selective media. In the course of these studies special attention has been given to investigation of *B*. *pseudomallei*-like strains, whose principal difference from causative agent of melioidosis lies in their avirulence to laboratory animals and enhanced biochemical activity as regards monosaccharides from the pentose group. Principal diagnostic features for these cultures and typical virulent strains of *B*. *pseudomallei* were identical. In 1998, these microorganisms have been assigned to the independent species of Burkholderia - *B*. *thailandensis.* At present this term has become generally acknowledged.

The most effective chemopreparations against pathogenic Burkholderia are tetracycline, fluoroquinalones, and combined sulfanylamides. Thus, it is mentioned in literature that in the treatment of glanders and melioidosis the best results have been obtained by using co-trimoxazole, and further in the order of decreasing effect - doxycycline, ciprofloxacin and ceftazidime (Iliukhin V.I., Senina T.V., Trushkina M.N., Shubnikova E.V., Antonov Yu.V., Andropova N.V. Problemy sootvetstviya antibiotikochuvstvitel'nosti in vitro I effektivnosti khimioterapii infektsii, vyzvanykh patogennymi burkkholderiiami (Problems of correspondence between antibiotic sensitivity in vitro and efficiency of chemotherapy of infections caused by pathogenic Burkholderia). - Antibiotiki i khimioterapiia, 2009.-No. 7-8.-p.19-23) (in Russian).

*Pseudomonas aeruginosa* is a species of straight or slightly curved Gram-negative bacteria which was separated into the independent species from the Pseudomonas genus on the basis of molecular genetic studies. Bacteria are motile by means of one or several flagellae. Aerobes. Widespread in nature in soil, in water, and on the plants.

*Pseudomonas aeruginosa* belong to causative agents of dangerous infections. They are exciting cause of various diseases in humans, such as sepsis, meningitis, osteomyelitis, arthritis, otitis, pneumonia, pleuritis, liver and brain abscesses, inflammation of urogenital system, and also causes alimentary toxicoinfection, while pyoinflammatory complications of operative wounds, burns, in turn, sometimes render treatment ineffective.

Infection arise as a result of contact with infected water, food, soil, as well as some objects, on which or within which *Pseudomonas aeruginosa* bacteria may be present. Spreading of causative agents is possible due to importation of sick animals, aquarium fishes, soil, water, as well as food products contaminated with these disease agents. The interest of researchers to *Pseudomonas aeruginosa* remains still alive due to a number of reasons.

All known species of Pseudomonas genus bacteria are characterized by high resistance to antibacterial agents, they all have in common high resistance to antibacterial preparations, including antibiotics. This natural resistance ensures their survivability in enviroinment and creates problems in disease treatment.

At present, infections comtrol worldwide assumes special character, since the expansion of international contacts, tourism and international trade create large opportunities for proliferation of pathogenic bacterial species belonging to the Pseudomonas genus from their natural endemic seats,. Recently, interest in diagnostics and treatment of said diseases has grown significantly in a number of countries in Europe and America, because features of causative agents of the Pseudomonas genus allows them to create relatively stable secondary seats of infection when such causative agents come to different climatic conditions. Moreover, it is noted that in humans the diseases caused by *Pseudomonas aeruginosa* often proceed in severe form and are hard to treat.

The most effective chemopreparations against pathogenic strains of *Pseudomonas aeruginosa* comprise antibiotics from the groups of carbapenems, cephalosporins, and penicillins. Thus, it is mentioned in literature that the greatest activity against *P. aeruginosa* of all antibiotics studied is displayed by meropenem, imipenem, cefepime, piperacillin, tazobactam, and amikacin (Goossens H. Susceptibility of multi-drug-resistant Pseudomonas aeruginosa in intensive care units: results from the European MYSTIC study group.-Clin. Microbiol. Infect.-2003.-9.-980-3).

*Pseudomonas aeruginosa* is one of the main causative agents of local and systemic pyoinflammatory processes during institutionalized treatment. The risk of development of infection caused by *Pseudomonas aeruginosa* increases substantially in patients with impairments of barrier systems and resistivity factors. Especially subject to risk of development of severe infections caused by *Pseudomonas aeruginosa* are patients weakened by mucoviscidosis, as well as those with neutropenia. In hospitals, *Pseudomonas aeruginosa* are discovered on utensils and articles, sanitary appliances, including washbasins, on urinals, catheters, may be found in intestine, in respiratory tract and on the skin of attending personnel, as well as in antiseptic solutions and in aquaeous solutions of medicaments.

Nosocomial pneumonia (also named hospital-acquired pneumonia) caused by bacteria of the *Pseudomonas aeruginosa* species, is one of infectious diseases which most commonly develop in hospitalized patients - by incidence it is ranked second among the nosocomial infections. Nosocomial pneumonia is a disease characterized by appearance of newly developed focal infiltrates on the lung X-ray 48 hours and more after hospitalization of the patient, in conjunction with clinical symptomatics confirming the infectious nature of the disease, while excluding infections latent on the moment of admission to the hospital.

Taking the above into account, it is clear that problem of treatment of nosocomial pneumonia caused by bacteria of the *Pseudomonas aeruginosa* species has attracted and continues to attract special attention from physicians. To study decamethoxine action against bacteria of the *Pseudomonas aeruginosa* species, the authors have conducted studies in order to determine the decamethoxine action against bacteria of the *Pseudomonas aeruginosa* species.

Decamethoxine is a chemical conpound with a chemical name 1,10-decamethylene-bis(N,N-dimethyl-menthoxycarbonylmethyl)ammonium dichloride, white crystalline powder with a faint odour, readily soluble in water, in chemical structure and antimicrobial action similar to ethonium.

It is noted in the literature that decamethoxine possesses the pronounced bactericidal action against staphylococci, streptococci, diphtheria bacillus, capsuliferous bacteria, and viruses, displays fungicidal activity toward yeasts, yeast-like fungi, causative agents of epidermophytosis, trichophytosis, microsporia, erythrasma, and some species of mold fungi (aspergilli, penicilli). It is used to treat impetiginous bacterial and fungal skin diseases (abscesses, purulent wounds, candidiasis et al.), microbial eczema, pyoinflammatory lesions of soft tissues (abscesses, carbuncles, phlegmons, furuncles, purulent wounds, felons, purulent conjunctivitis, gingivitis, periodontitis, tonsillitis, otitis) in cases with staphylococci and diphtheria bacilli carriage.

Analysis of the prior art publications concerning decamethoxine performed by the present authors has demonstrated the absence of data on the effects of decamethoxine on bacteria of the Burkholderia genus, and the absence of data on the effects of decamethoxine on bacteria of the *Pseudomonas aeruginosa* species, and data are also absent on the use of decamethoxine in the treatment of such diseases.

### Disclosure of the invention

The task of the technical solution lies in the use of decamethoxine as antimicrobial agent for a new purpose.

This task is solved by the use of decamethoxine as antimicrobial agent against bacteria of the Burkholderia genus or against bacteria of the *Pseudomonas aeruginosa* species.

Morover, the task is solved by the use of decamethoxine as antimicrobial agent against bacteria of *Burkholderia ambifaria, Burkholderia anthina, Burkholderia cenocepacia, Burkholderia cepacia, Burkholderia dolosa, Burkholderia gladioli, Burkholderia mallei, Burkholderia multivorans, Burkholderia pseudomallei, Burkholderia pyrrocinia, Burkholderia stabilis,* and *Burkholderia vietnamiensis* species.

Morover, the task is solved by the use of decamethoxine as antimicrobial agent against bacteria of the *Pseudomonas aeruginosa* species in the treatment of disease caused by bacteria of the *Pseudomonas aeruginosa* species, such as nosocomial pneumonia.

### The embodiment(s) of the invention

In order to study the effects of decamethoxine on bacteria of the Burkholderia genus, the present authors have conducted a study. The studies of decamethoxine action on bacteria of the Burkholderia genus and comparative studies of a reference antimicrobial agent, such as chlorhexidine bigluconate, have been conducted according to conventional serial dilutions method in solid nutritional medium and by agar diffusion method (well method). When using the serial dilutions method in solid nutritional medium, solution of decamethoxine and solution of chlorhexidine bigluconate were mixed with agar suitable for the given test strain in 1:9 ratio. For this purpose, to all vials filled with 13.5 ml of molten agar cooled to 45-48°C, 1.5 ml of test solution (with known concentration) was added, mixed and poured into Petri dish. 1.5 ml of broth was added to dish with control medium instead of test compound. Agar surface is divided into several sectors, each of which is seeded with a plaque of one of test strains of microorganisms. The results are read after 18-24 h incubation in thermostat at 37°C. MIC value of test preparation has been used to calculate its concentration retarding growth of microorganisms on agar surface (single colonies). MBC value of the preparation has been used to calculate its dilution corresponding to the absence of microbial colonies on the surface of nutritional medium.

Agar diffusion method (well method) is based on the ability of drug substances to penetrate into the depth of agar. Petri dishes placed on strictly horizontal surface are poured with two layers of solid nutritional medium. Lower layer consists of 10 ml of molten "hungry" Givental'-Ved'mina agar, and upper layer is a nutritional medium for corresponding test strain. After cooling the lower agar layer no more than six thin-walled cylinders (internal diameter of 6.0 + 0.1 mm, height 10.0 + 0.1 mm) are placed on it equidistantly from one another. Upper layer is poured around cylinders - 13.5 ml of cooled to 45-48°C molten agar mixed with seeding dose of test microorganism (1.5 ml of bacterial suspension with concentration corresponding to the microorganism species). After cooling down of the upper agar layer the cylinders are removed with sterile tweezers and 0.25-0.3 ml of test preparations and control samples are placed into the wells obtained. The results are read after 24 h by measuring growth inhibition zone, including diameter of the wells. Measurement is conducted accurate to 1 mm while aiming at the full absence of visible growth.

Bactericidal (MBC) action of the preparation in relation to museum strains and clinical isolates of bacteria was determined using killing-time curve. At that, bacterial cultures are grown in the presence of antimicrobial preparation and viability of microorganisms is determined at different time intervals by seeding on solid nutritional medium. Curve is plotted of bacterial death vs. time. To establish bactericidal activity, we have applied a micromethod utilizing sterile polystyrene 96-well plate. Daily test cultures of bacteria were used to prepare suspension of standard turbidity (10⁴ CFU/ml) in liquid nutritional medium, meat infusion broth (MIB). Suspension of test microorganism and test preparation in 1:1 ratio were added into each well and the mixture was cultured in thermostat at 37°C. The content of each well was used for plating on solid nutritional medium (sugar agar) at certain time intervals (1 hour, 3 hours, and 24 hours). The results were read after 24 hours of cultivation and the effects of test preparation were determined by the observed or absent growth of test strains on solid nutritional medium.

The results of studies were formalized by the activity in relation to bacteria of the Burkholderia genus into four kinds of activity: no activity; low activity; good activity; and high activity.

All experiments were accompanied by corresponding controls: medium sterility control; culture growth control in a medium without test preparation. The experiments were conducted in duplicate for each preparation concentration and test microorganisms culture in order to obtain reliable results.

Table 1 presents experimental data of decamethoxine and chlorhexidine bigluconate activity studies at different concentrations with various bacterial species of the Burkholderia genus.

**Table 1**

| Species of microorganism | Concentration of decamethoxine | | | | Concentration of chlorhexidine bigluconate | | | |
|---|---|---|---|---|---|---|---|---|
| | 0.02% | 0.05% | 0.1% | 0,2% | 0.02% | 0.05% | 0.1% | 0,2% |
| *Burkholderia cepacia* | + | ++ | +++ | +++ | - | - | + | + |
| *Burkholderia mallei* | + | ++ | +++ | +++ | - | - | + | + |
| *Burkholderia pseudomallei* | + | ++ | +++ | +++ | - | - | + | + |
| *Burkholderia cenocepacia* | + | ++ | +++ | +++ | + | + | + | + |
| *Burkholderia multivorans* | + | ++ | +++ | +++ | + | + | ++ | ++ |
| *Burkholderia vietnamiensis* | + | ++ | +++ | +++ | - | + | + | + |
| *Burkholderia dolosa* | + | ++ | +++ | +++ | + | + | ++ | ++ |
| *Burkholderia stabilis* | + | ++ | +++ | +++ | + | + | + | + |
| *Burkholderia ambifaria* | + | ++ | +++ | +++ | - | - | + | + |
| *Burkholderia anthina* | + | ++ | +++ | +++ | - | - | + | + |
| *Burkholderia pyrrocinia* | + | ++ | +++ | +++ | - | - | + | + |
| *Burkholderia gladioli* | + | ++ | +++ | +++ | + | + | + | ++ |

| | | | | | | | | |
|---|---|---|---|---|---|---|---|---|
| "-" no activity; "+" low activity; "++" good activity; "+++" high activity. | | | | | | | | |

To study effects of decamethoxine against infections caused by bacteria of the Burkholderia genus, treatment has been provided to a group of 40 patients with established non-nosocomial pneumonia aged 18 to 70 years (mean age 45 years), including 26 (75%) men and 14 (35%) women. Causative agent of non-nosocomial pneumonia in this group of patients is *Burkholderia cepacia.*

The most typical clinical implications in patients at the moment of hospitalization were: fever in 24 patients (60%), productive cough in 9 patients (22%) and underproductive cough in 31 (78%), with "strong" character of cough being determined in 22 patients (55%). Chest pain has been observed in 22 (56%) patients, including pronounced in 10 (45%) and moderate in 12 (55%); and dyspnea in 25 (63%) patients, pronounced in 10 (40%) and moderate in 15 (60%). Intoxication syndrome and sanitation more often manifest themself with such symptoms as weakness, sweating, and headache.

The diagnosis of pneumonia had been confirmed in all patients by X-ray study data in 2 projections (straight and side). According to the results of general clinical blood analysis, leukocytosis was found in 21 (52,5%) patients, at that in 18 (45%) cases stab cell shift to the left took place in leukogram. ESR acceleration before the beginning of treatment had been noted in 30 (75%) patients with pneumonia.

The treatment had been conducted using 0.02% solution of decamethoxine. The preparation was administered by ultrasonic inhalations method at 5-20 ml 1-2 times daily.

Positive dynamics has been noted in most patients: decrease in dyspnea and chest pain, and intensity of the intoxication syndrome. On days 3-5 considerable positive dynamics has been noted in evaluation of sputum character. Body temperature has been normalized in most patients (75%), features of respiratory failure have diminished. Dyspnea persisted in 30% of patients who have characterized it as "insignificant". Notwithstanding the persistent cough in 75% patients, its character has been changed from "strong" to "moderate" or "weak".

Positive auscultatory dynamics has been noted also by physical examination of patients with non-nosocomial pneumonia, fine moist rales were heard in 43% of cases. On days 7-10 disease features persisted in small number of patients. 14% of patients complainted of moderate cough.

Thus, positive effect after decamethoxine therapy has been noted in 41% of patients. Mean therapy time is 7 days.

Good tolerance to the prepration therapy has been observed in all patients. No patients had negative side effects that would make necessary to withdraw the preparation.

Similar studies have been conducted in other groups of patients using 0.05%, 0.1%, and 0.2% solutions of decamethoxine. The results of the therapy were sensibly positive. Most patients demonstrated good tolerance to these concentrations, and no adverse side effects were observed. Mean therapy time was 5 days.

The present authors have conducted studies to determine activity of decamethoxine against bacteria of the *Pseudomonas aeruginosa* species.

The studies to determine the effects of decamethoxine against bacteria of the *Pseudomonas aeruginosa* species have included patients with nosocomial pneumonia arising in hospital 48 hours or more after admission. The diagnosis of nosocomial pneumonia has been established on basis of following clinical criteria, such as appearance of new infiltrates in lung X-ray or progression of existing infiltrates, plus presence of any of such clinical symptoms: temperature above 38 °C, leukocytosis, purulent bronchial secretion. The studies have included patients with *P. aeruginosa* detected on plates seeded with sputum in titer quantities of >10⁵ colony-forming units in 1 ml (CFU/ml). Registered at the time of enlisting patients into the study were concomitant diseases, severity of lung pathological condition according to CPIS scale (Clinical Pulmonary Infection Score, a scale for clinical evaluation of lung infection), presence or absence of systemic inflammatory response syndrome. Condition of patients has been evaluated regularly by clinical symptoms, analyses of gas composition of arterial blood, severity of lung pathology condition by CPIS scale, and general and biochemical blood analysis.

Decamethoxine had been prescribed as 0.02 wt%, 0.05 wt%, and 0.1 wt% solutions. The introduction route of decamethoxine solutions was by inhalation, using Ulaizer Pro compressor inhalator.

Treatment of nosocomial pneumonia had been considered clinical and effective given the following criteria:
- fever decrease to <38 °C and purulent sputum decrease;
- infiltrates decrease in lung by X-ray;
- absence of septic shock or multiple organ failure.

Further are presented the results of studies of decamethoxine activity against bacteria of the *Pseudomonas aeruginosa* species. 50 patients 25-55 years of age with nosocomial pneumonia participated in the studies. Principal clinical symptoms observed in patients were: X-ray confirmation of focal infiltration of lung tissue (negative dynamics has distinguished X-ray pictures of chest organs by way of pronounced lung marking with substantial decrease in transparency of lung fields); acute fever (temperature >38 °C); cough with sputum; physical symptoms (focal crepitation and/or fine rales, harsh bronchial breathing, dullness on percussion), leukocytosis >10⁹/l and stab cell shifts (>10%).

Patients have been divided into three groups: first group had been prescribed 0.02 wt% solution of decamethoxine by inhalation, second group received 0.05 wt% solution of decamethoxine, and third group - 0.1 wt% solution of decamethoxine.

Inhalation therapy with solutions of decamethoxine lasted for 10 days. On therapy day 2 already positive dynamics had been noted by subjective and objective criteria: decreased dyspnea, decrease in body temperature, lowered inflammation markers level.

Examination of patients after the course of therapy has demonstrated that dyspnea and cough were disappearing, and laboratory characteristics indicating the presence of inflammatory process in the body, such as C-reactive protein plasma concentration, were in norm. Positive dynamics had been observed by control X-ray study as diminishing excessiveness of lung marking. On plating of the sputum performed on day 7 of therapy, only 10² CFU/ml *P. aeruginosa* had been isolated in titer.

Inhalations of 0.02 wt%, 0.05 wt%, and 0.1 wt% solutions of decamethoxine were well tolerated by patients: no bronchospasm or cough had been observed, as well as no increase in creatinine and urea levels.

To evaluate condition of patients during the studies, following clinical, laboratory and functional indices were used: estimation of lung pathology by CPIS scale, blood leukocytes level, plasma C-reactive protein (CRP) level; data on the dynamics of these indices in the group of patients receiving 0.02 wt% solution of decamethoxine are listed in Table 2, data on the dynamics of these indices in the group of patients receiving 0.05 wt% solution of decamethoxine are listed in Table 3, and data on the dynamics of these indices in the group of patients receiving 0.1 wt% solution of decamethoxine are listed in Table 4.

**Table 2**

| Indices (mean in group) | Pretreatment | Day 1 of treatment | Day 2 of treatment | Day 7 of treatment | Day 10 of treatment |
|---|---|---|---|---|---|
| Estimation by CPIS scale, score | 8 | - | 4 | 0 | 0 |
| Blood leukocytes, ×10⁹/l | 11,2 | - | 10,4 | 7,5 | 5 |
| Plasma CRP level, mg/l | 143 | - | 121 | 48 | 10 |

**Table 3**

| Indices (mean in group) | Pretreatment | Day 1 of treatment | Day 2 of treatment | Day 7 of treatment | Day 10 of treatment |
|---|---|---|---|---|---|
| Estimation by CPIS scale, score | 8 | - | 4 | 0 | 0 |
| Blood leukocytes, ×10⁹/l | 12,6 | - | 11,0 | 6,5 | 4 |
| Plasma CRP level, mg/l | 111 | - | 93 | 45 | 9 |

**Table 4**

| Indices (mean in group) | Pretreatment | Day 1 of treatment | Day 2 of treatment | Day 7 of treatment | Day 10 of treatment |
|---|---|---|---|---|---|
| Estimation by CPIS scale, score | 8 | - | 4 | 0 | 0 |
| Blood leukocytes, ×10⁹/l | 11,0 | - | 9,3 | 6,0 | 4 |
| Plasma CRP level, mg/l | 123 | - | 90 | 40 | 8 |

It is seen from the data in Tables 2 to 4 that in all three groups of patients on day 10 of therapy clinical, laboratory and functional indices were in norm, thus making it possible to conclude that the patients were cured.

Accordingly, it may be concluded that:
1. Decamethoxine displays good antimicrobial activity against bacteria of the Burkholderia genus or bacteria of the *Pseudomonas aeruginosa* species.
2. On increasing concentration of decamethoxine its antimicrobial activity against bacteria of the Burkholderia genus or bacteria of the *Pseudomonas aeruginosa* species is enhanced.
3. Decamethoxine exhibits bactericidal activity against all species of bacteria of the Burkholderia genus.

The technical result achieved by the present technical solution: new decamethoxine property found (antimicrobial action against bacteria of the Burkholderia genus or the *Pseudomonas aeruginosa* species) makes possible the development of more effective drugs for the treatment of diseases caused by bacteria of the Burkholderia genus or bacteria of the *Pseudomonas aeruginosa* species, in particular, for the treatment of nosocomial pneumonia, and adds to the arsenal of drugs for the treatment of diseases caused by bacteria of the Burkholderia genus or bacteria of the *Pseudomonas aeruginosa* species.

The exemplary embodiments of the technical solution provided herein illustrate the technical solution without limiting it.

## Claims

1. Use of decamethoxine as antimicrobial agent against bacteria of the Burkholderia genus or bacteria of the *Pseudomonas aeruginosa* species.

2. Use of decamethoxine according to claim 1, **characterized in that** the bacteria of the Burkholderia genus are bacteria of *Burkholderia ambifaria, Burkholderia anthina, Burkholderia cenocepacia, Burkholderia cepacia, Burkholderia dolosa, Burkholderia gladioli, Burkholderia mallei, Burkholderia multivorans, Burkholderia pseudomallei, Burkholderia pyrrocinia, Burkholderia stabilis,* and *Burkholderia vietnamiensis* species.

3. Use of decamethoxine according to claim 1, **characterized in that** the decamethoxine is used as antimicrobial agent against bacteria of the *Pseudomonas aeruginosa* species in the treatment of disease caused by bacteria of the *Pseudomonas aeruginosa* species, such as nosocomial pneumonia.
